# EUROPÄISCHE PATENTSCHRIFT

(11) **EP 1 699 749 B1**
(45) Veröffentlichungstag und Bekanntmachung des Hinweises auf die Patenterteilung: **09.09.2015**
(21) Anmeldenummer: 04804131.3
(22) Anmeldetag: 21.12.2004
(51) Int. Cl.: B01J 19/24, B01J 8/02, C07C 45/35, C07C 51/21

(54) **VERFAHREN ZUR HERSTELLUNG VON (METH)ACROLEIN UND/ODER (METH)ACRYLSÄURE DURCH HETEROGEN KATALYSIERTE PARTIALOXIDATION VON C3 UND/ODER C4-VORLÄ UFERVERBINDUNGEN**
METHOD FOR THE PRODUCTION OF (METH)ACROLEIN AND/OR (METH)ACRYLIC ACID BY MEANS OF HETEROGENEOUSLY CATALYZED PARTIAL OXIDATION OF C3 AND/OR C4 PRECURSOR COMPOUNDS
PROCEDE POUR PRODUIRE DE LA(METH)ACROLEINE ET/OU DE L'ACIDE (METH)ACRYLIQUE PAR OXYDATION PARTIELLE EN CATALYSE HETEROGENE DE COMPOSES PRECURSEURS C3 ET/OU C4

(30) Priorität: 23.12.2003 DE 10361456; 23.12.2003 US 531677 P
(43) Veröffentlichungstag der Anmeldung: 13.09.2006
(73) Patentinhaber: BASF SE, 67056 Ludwigshafen (DE)
(72) Erfinder: HECHLER, Claus, 67063 Ludwigshafen (DE); OLBERT, Gerhard, 69221 Dossenheim (DE); LÖWEN, Dietmar, 66987 Thaleischweiler-Fröschen (DE)
(86) Internationale Anmeldenummer: PCT/EP2004/014533
(87) Internationale Veröffentlichungsnummer: WO 2005/063676

(56) Entgegenhaltungen:
- WO-A-01/32301

## Beschreibung

Die Erfindung betrifft ein Verfahren zur Herstellung von (Meth)acrolein und/oder (Meth)acrylsäure durch Partialoxidation in der Gasphase von C₃- und/oder C₄-Vorläuferverbindungen in Gegenwart eines heterogenen partikelförmigen Katalysators in 1, 2 oder 3 Reaktionsstufen in einem Reaktor mit Thermoblechplattenmodulen.

Die abgekürzte Schreibweise (Meth)acrolein bezeichnet in bekannter Weise Acrolein und/oder Methacrolein. Analog wird die abgekürzte Schreibweise (Meth)acrylsäure für Acrylsäure und/oder Methacrylsäure verwendet.

Die Partialoxidation von C₃- und/oder C₄-Vorläuferverbindungen, wie Propylen, Propan, iso-Buten, iso-Butan, iso-Butanol, Methylether des i-Butanols, Acrolein oder Methacrolein (d.h. insbesondere von 3 oder 4 Kohlenstoffatomen enthaltenden Kohlenwasserstoffen) in der Gasphase wird bekanntermaßen in Gegenwart von heterogenen partikelförmigen Katalysatoren durchgeführt. Diese Umsetzungen sind stark exotherm, und werden bislang im großtechnischen Maßstab überwiegend in Rohrbündelreaktoren durchgeführt, mit Kontaktrohren, in denen der heterogene partikelförmige Katalysator eingebracht ist und durch die das gasförmige Reaktionsgemisch geleitet wird und wobei die freiwerdende Reaktionswärme indirekt, über einen Wärmeträger abgeführt wird, der im Zwischenraum zwischen den Kontaktrohren zirkuliert. Als Wärmeträger wird häufig eine Salzschmelze eingesetzt.

Die Reaktion kann ausgehend von einem Alkan in einer Reaktionsstufe zur Säure oder in einer ersten Stufe zum Aldehyd und in einer zweiten Stufe zur Säure durchgeführt werden. Eine alternative Durchführung kann in einer ersten von drei Stufen vom Alkan zum Olefin, in einer zweiten vom Olefin zum Aldehyd und in einer dritten vom Aldehyd zur Säure gehen. Ausgehend vom Olefin kann wiederum in zwei Stufen zunächst zum Aldehyd und dann zur Säure oxidiert werden oder auch in einer Stufe vom Olefin zur Säure oxidiert werden. Die Herstellung der Säure kann auch in einer Stufe ausgehend vom jeweiligen Aldehyd erfolgen. Als Aldehyd wird hierbei (Meth)acrolein verstanden und als Säure (Meth)acrylsäure.

Alternativ ist es auch möglich, die Reaktionswärme über einen Wärmeträger abzuführen, der durch plattenförmige Wärmeüberträger geleitet wird. Für plattenförmige Wärmeüberträger werden die Begriffe Wärmetauscherplatten, Wärmeübertragerplatten, Thermobleche, Thermoplatten oder Thermoblechplatten weitgehend synonym verwendet.

Wärmeübertragerplatten werden als überwiegend flächenförmige Gebilde definiert, die einen mit Zu- und Abführleitungen versehenen Innenraum mit geringer Dicke im Verhältnis zur Fläche aufweisen. Sie werden in der Regel aus Blechen, häufig aus Stahlblechen, hergestellt. Je nach Anwendungsfall, insbesondere den Eigenschaften des Reaktionsmediums sowie des Wärmeträgers, können jedoch spezielle, insbesondere korrosionsfeste, aber auch beschichtete Werkstoffe zum Einsatz kommen. Die Zu- bzw. Abführeinrichtungen für die Wärmeträger sind in der Regel an einander entgegengesetzten Enden der Wärmetauschplatten angeordnet. Als Wärmeträger kommen häufig Wasser, aber auch Diphyl® (Gemisch aus 70 bis 75 Gew.-% Diphenylether und 25 bis 30 Gew.-% Diphenyl) zum Einsatz, welche auch teilweise in einem Siedevorgang verdampfen; es ist auch der Einsatz anderer organischer Wärmeträger mit niedrigem Dampfdruck und auch ionischer Flüssigkeiten möglich.

Die Verwendung ionischer Flüssigkeiten als Wärmeträger ist in der DE-A 103 16 418 beschrieben. Bevorzugt sind ionische Flüssigkeiten, die ein Sulfat-, Phosphat-, Borat- oder Silikatanion enthalten. Besonders geeignet sind auch ionische Flüssigkeiten, die ein einwertiges Metall-Kation, insbesondere ein Alkalimetall-Kation, sowie ein weiteres Kation, insbesondere ein Imidazolium-Kation, enthalten. Vorteilhaft sind auch ionische Flüssigkeiten, die als Kation ein Imidazolium-, Pyridinium- oder Phosphonium-Kation enthalten.

Der Begriff Thermobleche oder Thermoblechplatten wird insbesondere für Wärmeübertragerplatten verwendet, deren einzelne, meistens zwei, Bleche durch Punkt- und/oder Rollschweißungen miteinander verbunden und häufig unter Verwendung hydraulischen Drucks plastisch unter Kissenbildung ausgeformt sind.

Die Begriffe Wärmetauscherplatte, Wärmeübertragerplatte, Thermobleche, Thermoplatte oder Thermoblechplatte werden vorliegend im Sinne der obigen Definition verwendet.

Reaktoren zur Durchführung von Partialoxidationen unter Verwendung von Thermoblechen sind beispielsweise aus DE-A 199 52 964 bekannt. Beschrieben ist die Anordnung eines Katalysators zur Durchführung von Partialoxidationen in einer Schüttung um Wärmeübertragerplatten in einem Reaktor. Das Reaktionsgemisch wird an einem Reaktorende dem Reaktorinnenraum zwischen den Wärmeübertragerplatten zugeführt und am entgegengesetzten Ende abgeführt und durchströmt somit den Zwischenraum zwischen den Wärmeübertragerplatten.

Die DE-C 197 54 185 beschreibt einen weiteren Reaktor mit indirekter Wärmeabführung über ein Kühlmedium, das durch Wärmeübertragerplatten strömt, wobei die Wärmeübertragerplatten als Thermobleche ausgebildet sind, die aus zumindest zwei Blechplatten aus Stahl bestehen, die an vorgegebenen Punkten unter Bildung von Strömungskanälen zusammengefügt sind.

Eine vorteilhafte Weiterbildung hiervon ist in DE-A 198 48 208 beschrieben, wonach Wärmeübertragerplatten, die als von einem Kühlmedium durchströmte Thermobleche ausgebildet sind, zu Plattenpaketen mit beispielsweise rechteckigem oder quadratischem Querschnitt zusammengefasst sind und die Plattenpakete eine so genannte Einhausung aufweisen. Das eingehauste Plattenpaket ist umfangseitig anpassungsfrei und folglich mit vorgegebenen Abständen zu der Innenwand des zylindrischen Reaktorbehälters eingesetzt. Die Freiflächen zwischen dem Plattenwärmeübertrager bzw. seiner Einhausung und der Behälterinnenwand sind im oberen und unteren Bereich der Einhausung mit Leitblechen abgedeckt, um den Bypass von Reaktionsmedium um die mit Katalysator gefüllten Kammern zu vermeiden.

Ein weiterer Reaktor mit Einrichtungen zur Abführung der Reaktionswärme in Form von Plattenwärmeübertragern ist in WO-A 01/85331 beschrieben. Der Reaktor von überwiegend zylindrischer Form enthält ein zusammenhängendes Katalysatorbett, in das ein Plattenwärmeübertrager eingebettet ist.

Aus DE-A 103 33 866 ist es bekannt, Probleme, die sich durch Deformationen aufgrund einseitiger hoher Belastung der Thermobleche bei zu großem Druckunterschied zwischen dem Reaktionsgemisch und der äußeren Umgebung ergeben, sowie mechanische Stabilitätsprobleme durch Verformung unter starker thermischer Beanspruchung, die auftreten können, wenn das Reaktionsgemisch unter Überdruck oder Unterdruck steht, zu vermeiden, indem ein Reaktor für Partialoxidationen eines fluiden Reaktionsgemisches in Gegenwart eines heterogenen partikelförmigen Katalysators zur Verfügung gestellt wird, mit
- einem oder mehreren quaderförmigen Thermoblechplattenmodulen, die jeweils aus zwei oder mehreren rechteckigen, parallel zueinander unter Freilassung jeweils eines Spaltes angeordneten Thermoblechplatten gebildet sind, der mit dem heterogenen partikelförmigen Katalysator befüllbar ist und der vom fluiden Reaktionsgemisch durchströmt wird, wobei die Reaktionswärme von einem Wärmeträger aufgenommen wird, der die Thermoblechplatten durchströmt und dabei zumindest teilweise verdampft, mit
- einer die Thermoblechplattenmodule druckentlastenden, dieselben vollständig umgebenden überwiegend zylinderförmigen Hülle, umfassend einen Zylindermantel und denselben an beiden Enden abschließenden Hauben und deren Längsachse parallel zur Ebene der Thermoblechplatten ausgerichtet ist, sowie mit
- einem oder mehreren Abdichtelementen, die dergestalt angeordnet sind, dass das gasförmige Reaktionsgemisch außer durch die von den Hauben begrenzten Reaktorinnenräume nur durch die Spalte strömt.

Es war demgegenüber Aufgabe der Erfindung, ein Verfahren zur Überwachung, Steuerung und/oder Regelung in einem Verfahren zur Herstellung von (Meth)acrolein und/oder (Meth)acrylsäure durch Partialoxidation von C₃- und/oder C₄-Vorläuferverbindungen in der Gasphase zur Verfügung zu stellen.

Entsprechend wurde ein Verfahren zur Herstellung von (Meth)acrolein und/oder (Meth)acrylsäure durch Partialoxidation von C₃- und/oder C₄-Vorläuferverbindungen in der Gasphase in Gegenwart eines heterogenen partikelförmigen Katalysators gefunden, in einem Reaktor mit zwei oder mehreren, vertikal, parallel zueinander unter Freilassung jeweils eines Spaltes 2 angeordneten Thermoblechplatten 1, wobei in den Spalten 2 der heterogene partikelförmige Katalysator eingebracht ist und das gasförmige Reaktionsgemisch durch die Spalte 2 geleitet wird, das dadurch gekennzeichnet ist, dass man das Verfahren überwacht, steuert und/oder regelt, indem man als Überwachungs-, Steuerungs- und/oder Regelgröße einen oder mehrere Temperaturwerte wählt, die man in einem oder mehreren Spalten 2, an einer oder mehreren Messstellen, die über die Höhe jedes Spaltes 2 verteilt angeordnet sind, misst, unter Nutzung einer Vorrichtung mit einer Hülse (3), die im Spalt (2) angeordnet ist, die außerhalb des Reaktors mündet und die jeweils einen Temperaturmesseinsatz (4) umhüllt, der ein Multithermoelement ist.

Vorzugsweise wählt man zusätzlich als weitere Überwachungs-, Steuerungs- und/oder Regelgröße die Zusammensetzung des fluiden Reaktionsgemisches in einem oder mehreren Spalten, die man an einer oder mehreren Messstellen, die über die Höhe jedes Spaltes verteilt angeordnet sind, bestimmt.

Für die Bestimmung der Betriebsbedingungen von Reaktoren ist die Kenntnis des Temperaturfelds im Katalysatorbett von wesentlicher Bedeutung. Dies betrifft die örtliche Verteilung der Temperatur, wie auch zum Beispiel die Höhe und Lage des Temperaturmaximums (Hot-Spot). Auch der Temperaturverlauf entlang des Strömungsweges des Reaktionsmediums kann für die Steuerung und Regelung des Reaktionssystems wesentlich sein.

Neben dem stationären Betrieb müssen auch das An- oder Abfahren oder etwa zeitlich veränderliche Rahmenbedingungen des Betriebs auch über längere Zeiträume zum Beispiel eine Veränderung der Katalysatoraktivität (Desaktivierung) beherrscht werden. Auf Grundlage gemessener Temperaturen kann zum Beispiel ein sicherer Betrieb gewährleistet, aber auch der jeweils bevorzugte, optimale Betriebszustand angesteuert und aufrechterhalten werden. Es sind Rückschlüsse auf die günstigste Betriebsweise zum Beispiel bezüglich E-duktzusammensetzung und Eduktmengenstrom, aber auch Kühltemperatur und Kühlmediumdurchsatz möglich. Darüber hinaus kann durch zusätzliche Konzentrationsmessung in der Katalysatorschüttung der stoffliche Verlauf der Reaktion verfolgt werden und zum Beispiel auch die Reaktionskinetik unter Betriebsbedingungen bestimmt werden. Beispielsweise kann auch das Desaktivierungsverhalten des Katalysators anhand von Konzentrationsverläufen im Verlauf der Durchströmung insbesondere zusammen mit Temperaturprofilen charakterisiert werden, was zur vorteilhaften Reaktionsführung mit geringer Nebenproduktbildung auch angepasst an die Gaslast oder aber auch zur Verbesserung des Katalysators und des Reaktordesigns verwertet werden kann.

Bei Rohrbündelreaktoren ist es nach dem Stand der Technik erforderlich, beim Einsatz von Temperaturmesshülsen oder Temperaturmesseinsätzen in die Katalysatorschüttung speziell angefertigte Rohre mit vergrößertem Innendurchmesser zu verwenden, um in diesen Rohren einen mit den übrigen, normalen Reaktionsrohren gleichwertigen Reaktionsablauf und somit eine repräsentative Temperaturmessung zu ermöglichen.

Die Erfinder haben erkannt, dass es möglich ist, den Temperaturverlauf im partikelförmigen Katalysator, der in den Spalt zwischen zwei Thermoblechplatten eingebracht ist, über die Höhe desselben, das heißt den Temperaturverlauf entlang des Strömungswegs, und weiterhin auch den Konzentrationsverlauf über die Höhe des Katalysators, das heißt den Konzentrationsverlaufs entlang des Strömungswegs zu bestimmen, ohne Störung des Prozesses durch den Messvorgang selbst.

Reaktoren mit Thermoblechplatten wurden bereits vorstehend beschrieben.

Die Thermoblechplatten sind aus vorzugsweise korrosionsfreien Werkstoffen, insbesondere aus Edelstahl, beispielsweise mit der Werkstoffnummer 1.4541 bzw. 1.4404, 1.4571 bzw. 1.4406, 1.4539 aber auch 1.4547 und 1.4301 oder aus anderen legierten Stählen, gefertigt.

Die Materialstärke der hierfür eingesetzten Bleche kann zwischen 1 und 4 mm, 1,5 und 3 mm, aber auch zwischen 2 und 2,5 mm, oder zu 2,5 mm gewählt werden.

In der Regel werden zwei rechteckige Bleche an ihren Längs- und Stirnseiten zu einer Thermoblechplatte verbunden, wobei eine Rollnaht oder seitliches Zuschweißen oder eine Kombination von beidem möglich ist, so dass der Raum, in dem sich später der Wärmeträger befindet, allseitig dicht ist. Vorteilhaft wird der Rand der Thermoblechplatten an oder schon in der seitlichen Rollnaht der Längskante abgetrennt, damit der schlecht oder nicht gekühlte Randbereich, in dem meist auch Katalysator eingebracht ist, eine möglichst geringe geometrische Ausdehnung hat.

Über die Rechteckfläche verteilt werden die Bleche miteinander durch Punktschweißung verbunden. Auch eine zumindest teilweise Verbindung durch gerade oder auch gebogene und auch kreisförmige Rollnähte ist möglich. Auch die Unterteilung des vom Wärmeträger durchströmten Volumens in mehrere getrennte Bereiche durch zusätzliche Rollnähte ist möglich.

Eine Möglichkeit der Anordnung der Schweißpunkte auf den Thermoblechplatten ist in Reihen mit äquidistanten Punktabständen von 30 bis 80 mm oder auch 35 bis 70 mm, wobei auch Abstände von 40 bis 60 mm möglich sind, wobei eine weitere Ausführungsform Abstände von 45 bis 50 mm und auch 46 bis 48 mm ist. Typischerweise variieren die Punktabstände fertigungsbedingt bis zu ± 1 mm und die Schweißpunkte unmittelbar benachbarter Reihen sind in Längsrichtung der Platten gesehen, jeweils um einen halben Schweißpunktabstand versetzt angeordnet. Die Reihen der Punktschweißungen in Längsrichtung der Platten können äquidistant mit Abständen von 5 bis 50 mm, aber auch von 8 bis 25 mm, wobei auch Abstände von 10 bis 20 mm und auch 12 bis 14 mm, eingesetzt werden. Weiterhin sind auch dem Anwendungsfall angepasste Paarungen der genannten Schweißpunktabstände und Reihenabstände möglich. Die Reihenabstände können in einem definierten geometrischen Zusammenhang zum Punktabstand, typisch ¼ der Punktabstände oder etwas geringer sein, so dass sich eine definiert gleichmäßige Aufweitung der Thermobleche bei der Herstellung ergibt. Den vorgegebenen Schweißpunkt- und Reihenabständen ist eine definierte Anzahl von Schweißpunkten je Plattenoberflächeneinheit zugeordnet, mögliche Werte sind 200 bis 3000, typische Werte 1400 bis 2600 Schweißpunkte je m² Plattenoberfläche. Vorteilhaft liegen 20 bis 35 Schweißpunkte in einem rechteckigen Oberflächenteilbereich von 5 x Schweißpunktabstand und 5 x Reihenabstand.

Die Breite der Thermoblechplatten ist im Wesentlichen fertigungstechnisch begrenzt und kann zwischen 100 und 2500 mm, oder auch zwischen 500 und 1500 mm, liegen. Die Länge der Thermoblechplatten ist abhängig von der Reaktion, insbesondere vom Temperaturprofil der Reaktion, und kann zwischen 500 und 7000 mm, oder auch zwischen 3000 und 4000 mm liegen.

Jeweils zwei oder mehrere Thermoblechplatten sind parallel und beabstandet zueinander, unter Bildung eines Thermoblechplattenmoduls, angeordnet. Dadurch entstehen zwischen unmittelbar benachbarten Blechplatten schachtartige Spalte, die an den engsten Stellen des Plattenabstandes beispielsweise eine Breite zwischen 8 und 150 mm, aber auch 10 bis 100 mm aufweisen. Eine mögliche Ausführung sind auch Breiten von 12 bis 50 mm oder aber 14 bis 25 mm, wobei auch 16 bis 20 mm gewählt werden können. Es wurde auch schon ein Spaltabstand von 17 mm erprobt.

Zwischen den einzelnen Thermoblechplatten eines Thermoblechplattenmoduls können, z.B. bei großflächigen Platten, zusätzlich Distanzhalter eingebaut werden, um Verformungen vorzubeugen, welche Plattenabstand oder -position verändern können. Zum Einbau dieser Distanzhalter können Teilbereiche der Bleche durch zum Beispiel kreisförmige Rollnähte oder Schweißpunkte größeren Durchmessers vom Durchflussbereich des Wärmeträgers abgetrennt werden, um in deren Mitte dort beispielsweise Löcher für stabförmige Distanzhalter, die verschraubt oder verschweißt sein können, in die Platten einbringen zu können.

Die Spalte zwischen den einzelnen Platten können gleichen Abstand besitzen, bei Erfordernis können die Spalte aber auch unterschiedlich breit sein, wenn die Reaktion dies zulässt oder die gewünschte Reaktion es erfordert, oder apparative oder kühltechnische Vorteile erzielt werden können.

Die mit Katalysatorpartikeln gefüllten Spalte eines Thermoblechplattenmoduls können gegeneinander gedichtet, z.B. dichtgeschweißt sein oder auch prozessseitig zueinander Verbindung besitzen.

Zur Einstellung des gewünschten Spaltabstandes beim Zusammenfügen der einzelnen Thermoblechplatten zu einem Modul werden die Platten in ihrer Position und im Abstand fixiert.

Die Schweißpunkte unmittelbar benachbarter Thermoblechplatten können sich gegenüberliegen oder versetzt zueinander sein.

Gegenstand der Erfindung ist weiterhin eine Vorrichtung zur Durchführung des vorstehend beschriebenen Verfahrens, gekennzeichnet durch eine Hülse, die im Spalt zwischen zwei Thermoblechplatten, vorzugsweise in Längsrichtung angeordnet ist und außerhalb des Reaktors mündet und die einen Temperaturmesseinsatz, zum Beispiel ein oder mehrere Thermoelemente mit einer oder mehrerer Messstellen umhüllt.

Vorzugsweise sind die Thermoblechplatten in
- einem oder mehreren quaderförmigen Thermoblechplattenmodulen angeordnet, die jeweils aus zwei oder mehreren rechteckigen, parallel zueinander unter Freilassung jeweils eines Spaltes angeordneten Thermoblechplatten gebildet sind, wobei
- die Thermoblechplattenmodule mit einer druckentlastenden, überwiegend zylinderförmigen Hülle, umfassend einen Zylindermantel und denselben an beiden Enden abschließenden Hauben und deren Längsachse parallel zur Ebene der Thermoblechplatten ausgerichtet ist, vollständig umgeben sind, wobei
- ein oder mehrere Abdichtelemente dergestalt angeordnet sind, dass das gasförmige Reaktionsgemisch außer durch die von den Hauben begrenzten Reaktorinnenräume nur durch die Spalte strömt und wobei
- jedes Thermoblechplattenmodul mit einem oder mehreren voneinander unabhängigen Temperaturmesseinsätzen, bevorzugt mit zwei oder drei, besonders bevorzugt mit drei Temperaturmesseinsätzen ausgestattet ist.

Indem jedes Thermoblechplattenmodul mit jeweils mindestens einem unabhängigen Temperaturmesseinsatz ausgestattet ist, kann jedes Thermoblechplattenmodul einzeln beurteilt und überwacht werden. Vorteilhaft ist es, für jedes Thermoblechplattenmodul mehre als einen Temperaturmesseinsatz vorzusehen, so dass bei Ausfall eines einzelnen Temperaturmesseinsatzes dennoch der sichere Betrieb gewährleistet ist. Beim Einsatz von jeweils drei Temperaturmesseinsätzen pro Thermoblechplattenmodul ist es möglich, den sicheren Betrieb bei Prüfung, Wartung oder Ausfall eines Temperaturmesseinsatzes aufrechtzuerhalten, insbesondere auch dann, wenn die Temperatursignale funktionell in einer Schutzschaltung genutzt werden.

Die Hülse ist ein bevorzugt metallisches Rohr, insbesondere mit einem Außendurchmesser im Bereich von 4 bis 15, insbesondere von 6 bis 10 mm, häufig von 6 bis 8 mm und weiter bevorzugt mit einer Wandstärke von 0,8 bis 1,5 mm, bevorzugt von 1 mm. Es kommen für die Hülse prinzipiell die gleichen Werkstoffe in Frage, die für die Thermoblechplatten eingesetzt werden können, wobei Hülse und Thermoblechplatten nicht aus demselben Werkstoff sein müssen. Es können als Hülse auch Nicht-Eisen-Werkstoffe zum Einsatz kommen. Bei Rohrbündelreaktoren ist es nach dem Stand der Technik erforderlich, beim Einsatz von Temperaturmesshülsen oder Temperaturmesseinsätzen in der Katalysatorschüttung speziell angefertigte Rohre mit vergrößertem Innendurchmesser zu verwenden, um in diesen Rohren einen mit den übrigen, normalen Reaktionsrohren gleichwertigen Reaktionsablauf und somit eine repräsentative Temperaturmessung zu ermöglichen.

Während es bei der üblichen Anordnung von Hülsen zur Aufnahme von Messelementen in Reaktionsrohren, zentrisch, in Längsachse derselben, zu einer starken Verfälschung des Strömungs- und Temperaturprofils gegenüber Reaktionsrohren ohne eingebaute Hülsen kommt, und daher besondere Ausgestaltungen des Reaktionsrohres, der Katalysatorfüllung und auch der Hülse, beispielsweise mit unterschiedlicher Wandstärke über ihrem Querschnitt oder besondere Anordnungen der Hülse im Kontaktrohr erforderlich sind, wie in DE-A 101 10 847 beschrieben, wurde überraschend gefunden, dass es bei Reaktoren mit Thermoblechplatten zur Messung des Temperaturprofils im Katalysatorbett in den Spalten zwischen den Thermoblechplatten derartiger spezieller Anordnungen nicht zwingend bedarf.

Es ist lediglich erforderlich, den Temperaturmesseinsatz selbst oder die Hülse, die den Temperaturmesseinsatz umhüllt, im Spalt, bevorzugt in Längsrichtung zwischen zwei Thermoblechplatten anzuordnen.

Der Abstand des Temperaturmesseinsatzes oder der Hülse zu den beiden Thermoblechplatten kann dabei vorzugsweise jeweils gleich sein, das heißt der Temperaturmesseinsatz ist in einer Ausführungsform mittig im Spalt angeordnet.

Zur Einführung der Hülse in den Spalt zwischen den Thermoblechplatten ist es besonders vorteilhaft, wenn die Thermoblechplatten jeweils gleiche Schweißpunktmuster aufweisen und die Schweißpunkte benachbarter Thermoblechplatten einander gegenüberliegen.

Die Hülsen können außerhalb des Reaktors sowohl oberhalb als auch unterhalb desselben münden. In einer bevorzugten Ausführungsform ist es möglich, dass die Hülsen sowohl oberhalb als auch unterhalb des Reaktors münden. Dabei kann der Temperaturmesseinsatz kontinuierlich in der Hülse verschoben werden, so dass eine kontinuierliche Abbildung des Temperaturprofils bestimmt werden kann, nicht nur diskrete Temperaturmesswerte. Hierfür kann ein einzelnes Messelement, vorteilhaft aber auch ein Mehrfachmesselement, besonders vorteilhaft mit äquidistanten Messabständen verwendet werden, da der notwendige Verschiebeweg zur lückenlosen Messung des Temperaturprofils dann nur einen Messstellenabstand beträgt.

Die Hülsen können nahtlos durch die äußere Reaktorummantelung geführt werden oder auch Verbindungselemente im Bereich oberhalb der katalysatorgefüllten Thermoplattenmodule, bzw. bei Einführung von unten unterhalb der Thermoblechplattenmodule aufweisen. In einer besonders vorteilhaften Variante sind die Hülsen im Reaktorinnenraum mit Trennstellen versehen, die insbesondere als Schneid- oder Klemmringverbindung ausgeführt sind, so dass die Montage erheblich erleichtert ist.

Der Temperaturmesseinsatz weist in der Regel mehrere, über seine Länge und somit über die Höhe des Spaltes vereilt angeordnete Messstellen auf. Als Temperaturmesseinsätze kommen vorzugsweise Vielfach-Messeinsätze (so genannte Multithermoelemente) in Frage, es können aber auch alle andren, insbesondere physikalischen Temperaturmessprinzipien wie Platin-Widerstandsthermometer, beispielsweise PT-100 oder PT-1000, Widerstandsthermometer oder Halbleitersensoren verwendet werden. Es kommen je nach Einsatztemperatur alle in DIN43710 und DIN EN 60584 beschriebenen Thermoelemente in Frage, vorzugsweise Thermoelemente des Typs K nach DIN EN 60584.

Die verteilt angeordneten Messstellen können äquidistant angeordnet sein, besonders vorteilhaft jedoch in Reaktorbereichen mit zu erwartendem Temperaturextrema und/oder besonders großer Temperaturgradienten mit geringerem Abstand zueinander und in den übrigen Reaktorbereichen mit größerem Abstand zueinander.

Vorteilhaft weist der Temperaturmesseinsatz 5 bis 60 Messstellen, bevorzugt 10 bis 50 Messstellen, besonders bevorzugt 15 bis 40 Messstellen und weiter bevorzugt 20 bis 30 Messstellen auf.

In einer bevorzugten Ausführungsform weist der Temperaturmesseinsatz 20 Messstellen und einen Außendurchmesser von etwa 3,8 mm auf, so dass der Temperaturmesseinsatz in einer Hülse mit einem Außendurchmesser von 6 mm oder von 1/4 Zoll und einem Innendurchmesser von 4 mm oder von 5/32 Zoll montiert werden kann.

In einer weiteren bevorzugten Ausführungsform weist der Temperaturmesseinsatz 40 Messstellen auf und einen Außendurchmesser von etwa 2,5 mm, so dass er in einer Hülse mit einem Außendurchmesser von 5 mm oder von 3/16 Zoll und einem Innendurchmesser von 3 mm oder von 1/8 Zoll montiert werden kann.

In einer Ausführungsform kann die Hülse, die das Thermoelement umhüllt, an der seitlichen Begrenzung des Spaltes zwischen zwei Thermoblechplatten angeordnet sein. Um eine Messverfälschung zu vermeiden, ist hierbei bevorzugt ein Isolierkörper zwischen der seitlichen Begrenzung des Spaltes und der Hülse vorzusehen, so dass auch am Rand der Schüttung ein repräsentatives Temperatursignal erfasst werden kann. Besonders vorteilhaft ist hierbei, dass die Hülse fest im Spalt eingebaut ist und bleiben kann und nicht zusammen mit der Katalysatorfüllung ein- bzw. ausgebaut werden muss. Die Hülse kann in diesem Fall auch mit nicht-zylindrischer Geometrie, beispielsweise mit einem Quadrat- oder Halbkreisquerschnitt ausgeführt sein.

Es ist darüber hinaus auch möglich, die Hülse, die das Thermoelement umhüllt, horizontal im Spalt zwischen zwei Thermoblechplatten anzuordnen. Dadurch kann der Temperaturverlauf über den Querschnitt des Spaltes bestimmt werden.

In einer weiteren, bevorzugten Ausführungsform der erfindungsgemäßen Vorrichtung ist zusätzlich zu der vorstehend beschriebenen Hülse mit Temperaturmessung in einem oder mehreren Spalten jeweils eine Hülse vorgesehen, die Perforationen aufweist sowie mindestens ein Probenahmeröhrchen zum Einführen in das Innere der Hülse, welches dort dergestalt angeordnet ist, dass das gasförmige Reaktionsgemisch über die Perforationen in der Hülse in das Innere des Probenahmeröhrchen einströmt und aus dem Probenahmeröhrchen nach außerhalb des Reaktors abgezogen und analysiert wird.

Als Hülse wird in der Regel ein metallisches Rohr, bevorzugt mit einem Außendurchmesser im Bereich von 5 bis 15, insbesondere von 8 bis 10 mm und einer Wandstärke von bevorzugt 1 mm, eingesetzt. Die Hülse weist erfindungsgemäß Perforationen auf, das heißt Öffnungen zum Reaktionsraum hin" wobei dieselben grundsätzlich bezüglich der geometrischen Form nicht eingeschränkt sind. Bevorzugt sind die Öffnungen jedoch kreisförmig ausgebildet. Insbesondere ist auch eine schlitzförmige Ausführung mit Anordnung der Schlitze in Längsrichtung des Probenahmeröhrchens, möglich. Die Perforationen weisen bevorzugt eine Gesamtoberfläche von 1 bis 50 %, bevorzugt von 1 bis 10 %, bezogen auf die gesamte Mantelfläche der Hülse auf. Sie dienen dazu, das gasförmige Reaktionsgemisch in die Hülse einströmen zu lassen, und somit in das im Innern der Hülse angeordnete Probenahmeröhrchen über die Öffnung desselben zu gelangen. Die aus dem Probenahmeröhrchen außerhalb des Reaktors abgezogene Probe kann zum Beispiel mit der vorhandenen Betriebsanalytik analysiert werden. Es ist gleichermaßen möglich, Proben kontinuierlich oder in bestimmten Zeitabständen abzuziehen und zu analysieren.

Das Entnehmen der Proben kann hierbei durch den Eigendruck des Reaktionssystems über ein Regelventil oder Überströmeinrichtung oder aber mittels einer Pumpe bzw. Verdichters oder eines Strahlers/Ejektors erfolgen, wobei die Probe in ein System mit Atmosphärendruck aber auch Unter- oder Überdruck zur Atmosphäre eingeleitet werden kann. Vorzugsweise ist das Analysesystem, in welches die Probe eingeleitet wird zur Erhöhung der Messgenauigkeit auf konstanten Druck eingeregelt.

In einer bevorzugten Ausgestaltung ist die perforierte Hülse mittig im Spalt angeordnet. Bei dieser Anordnung ist die Symmetrie des Strömungsbildes im Spalt besonders wenig gestört. Der Einbau kann hierbei vertikal von oben oder unten erfolgen, wobei der Einbau vorzugsweise von derselben Seite des Reaktors erfolgt, wie die Zuführung des fluiden Reaktionsgemisches.

In der Ausführungsvariante, in der sowohl der Einbau der Hülsen sowie die Zuführung des fluiden Reaktionsgemisches jeweils von oben in den Reaktor erfolgen, weisen die Hülsen vorteilhaft lediglich im oberen Bereich des Spaltes, insbesondere bis etwa zur Mitte desselben, mit Perforationen versehen. Da sich das Probenahmeröhrchen nur im oberen Bereich der Hülse, bis zu der Stelle, an der die Probe zwecks Bestimmung ihrer Zusammensetzung über die Öffnung aufgenommen wird, erstreckt, würde der darunter angeordnete, leere Bereich der Hülse ansonsten einen Bypass für das Reaktionsgemisch darstellen. Dies wird verhindert, indem Perforationen in der Hülse nur im oberen Bereich des Spaltes vorgesehen werden.

Analog ist es möglich, dass der Einbau der Hülsen sowie die Zuführung des fluiden Reaktionsgemisches in den Reaktor jeweils von unten erfolgen und dass bevorzugt durch die Thermoblechplatten ein Wärmeträger geleitet wird, der unter Reaktionsbedingungen partiell oder vollständig siedet.

Bevorzugt kann das Probenahmeröhrchen mit der Hülse fest verbunden sein, dergestalt, dass die Öffnung des Probenahmeröhrchens unmittelbar an einer Perforation der Hülse angeordnet ist, die Öffnungen von Probenahmeröhrchen und Hülse sich somit überlagern.

In einer weiteren bevorzugten Ausgestaltung ist das Probenahmeröhrchen drehbar in der perforierten Hülse angeordnet und weist mindestens zwei, über seine Mantelfläche versetzt angeordnete Öffnungen auf, dergestalt, dass das gasförmige Reaktionsgemisch stets nur über eine der Öffnungen in das Probenahmeröhrchen einströmt. Bevorzugt sind die Öffnungen des Probenahmeröhrchens als Schlitze in Längsrichtung desselben angeordnet, wodurch mehr Spielraum beim Anpassen der Öffnungen von Hülse und Probenahmeröhrchen zur Verfügung steht.

Durch diese Ausgestaltung können mittels eines einzigen Probenahmeröhrchens Proben von mehreren Stellen, die über die Höhe des Spaltes verteilt angeordnet sind, entnommen werden.

In einer weiteren bevorzugten Variante weist jedes Probenahmeröhrchen mindestens zwei, bevorzugt zwei bis vier voneinander getrennte Kammern auf, mit jeweils einer Öffnung, in die das gasförmige Reaktionsgemisch über die Perforationen der Hülse einströmt und wobei das gasförmige Reaktionsgemisch aus jeder Kammer getrennt abgezogen und analysiert wird. Die Kammern können dabei nebeneinander oder konzentrisch zueinander angeordnet sein.

Durch Ausbildung von zwei oder mehreren voneinander getrennten Kammern in den Probenahmeröhrchen wird die Zahl der Messstellen, an denen Proben des fluiden Reaktionsgemisches abgezogen werden können, erhöht.

Besonders bevorzugt ist die Ausführungsvariante, in der ein Probenahmeröhrchen mit mehreren Kammern vorgesehen ist, das zusätzlich um seine Längsachse drehbar angeordnet ist. Dadurch können für jede Kammer zwei oder mehrere, bevorzugt vier gegeneinander versetzte Schlitze zur Aufnahme des gasförmigen Reaktionsgemisches angeordnet sein, wobei das gasförmige Reaktionsgemisch in jede Kammer stets jeweils nur über eine Öffnung einströmt. Durch diese Ausgestaltung wird die Zahl der Messstellen für die Zusammensetzung des gasförmigen Reaktionsgemisches weiter erhöht.

In einer weiteren bevorzugten Ausführungsvariante sind zwei oder mehrere Probenahmeröhrchen vorgesehen, die jeweils mit der Hülse fest verbunden sind, dergestalt, dass die Öffnung jedes Probenahmeröhrchens unmittelbar an einer Perforation der Hülse angeordnet ist und wobei die einzelnen Probenahmeröhrchen auf jeweils unterschiedlicher Höhe im Spalt münden. Darüber hinaus ist es auch möglich, die Hülse selbst als Probenahmeröhrchen auszugestalten, indem lediglich an den Stellen, an denen eine direkte Verbindung mit jeweils einem Probenahmeröhrchen besteht, Perforationen vorgesehen sind und darüber hinaus auf einer von der Mündung der Probenahmeröhrchen verschiedenen Stelle eine einzige weitere Perforation in der Hülse vorgesehen ist, über die gasförmiges Reaktionsgemisch einströmt.

Durch das erfindungsgemäße Verfahren ist somit eine genaue Kenntnis des tatsächlichen Reaktionsgeschehens und der realen Temperaturen, bevorzugt auch der für den Hot-Spot maßgeblichen Temperatur in einfacher Weise, unter Nutzung der vorhandenen Betriebsanalytik, möglich. Dadurch kann wesentlich näher an der Belastungsgrenze des Katalysators gefahren werden, der Katalysator kann somit besser ausgenutzt werden, wobei gleichzeitig Beschädigungen durch unerwünscht starke Hot Spot-Bildung vermieden werden. Weiterhin kann in Kenntnis des tatsächlichen Reaktionsgeschehens die Katalysatoraktivität räumlich im Spalt unterschiedlich, angepasst an das tatsächliche Reaktionsgeschehen, ausgestaltet werden. Dadurch wird der Katalysator geschont, insbesondere in den thermisch stärker belasteten Bereichen, und somit seine Alterungsgrenze erhöht.

Darüber hinaus kann der Reaktor zur Herstellung von (Meth)acrolein und/oder (Meth)acrylsäure wesentlich gleichförmiger betrieben werden, wodurch die Gesamtselektivität der darin stattfindenden Reaktionen positiv beeinflusst wird. Weiterhin kann durch Anpassung der Katalysatoraktivität an das tatsächliche Reaktionsgeschehen die benötigte Menge an Wärmeträger reduziert werden.

Die Erfindung wird im Folgenden anhand einer Zeichnung näher erläutert.

Es zeigen im Einzelnen:
- Figur 1: einen Ausschnitt aus einem Reaktor mit Thermoblechplatten mit mittig angeordneter Hülse zur Aufnahme eines Thermoelementes, im Längsschnitt, mit Querschnittsdarstellung in Figur 1A,
- Figur 2: einen Ausschnitt durch eine weitere Ausführungsform mit seitlich angeordneter Hülse, im Längsschnitt, mit Querschnittsdarstellung in Figur 2A,
- Figur 3: eine weitere Ausführungsform mit horizontal im Spalt angeordneter Hülse, im Längsschnitt, mit Querschnittsdarstellung in Figur 3A und Detaildarstellung in Figur 3B,
- Figur 4: einen Ausschnitt aus einer weiteren Ausführungsform mit einer Hülse mit Perforationen und Probenahmeröhrchen, im Längsschnitt, mit Querschnittsdarstellung in Figur 4A und
- Figur 5: die schematische Darstellung für den Einbau einer erfindungsgemäßen Hülse in ein Thermoblechplattenmodul.
- Figur 6: schematisch bevorzugte Schweißpunktverteilungen auf der Oberfläche von Thermoblechplatten.

In den Figuren bezeichnen gleiche Bezugszeichen gleiche oder entsprechende Merkmale.

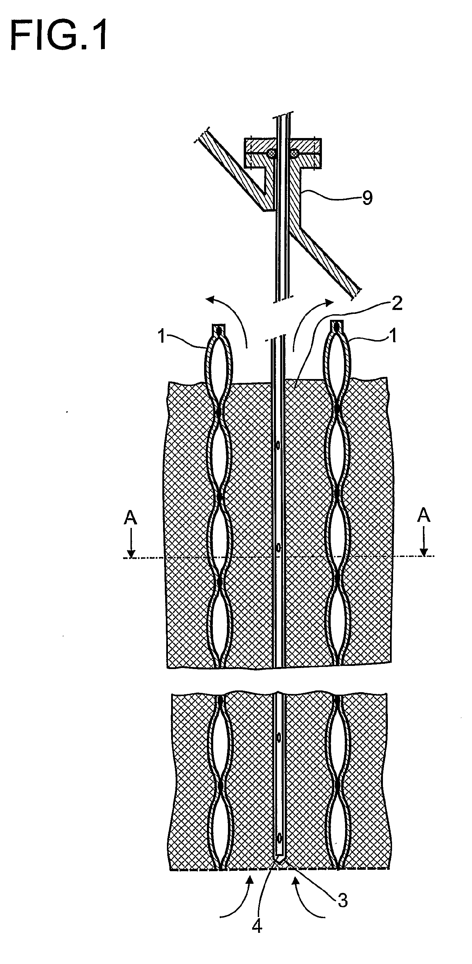

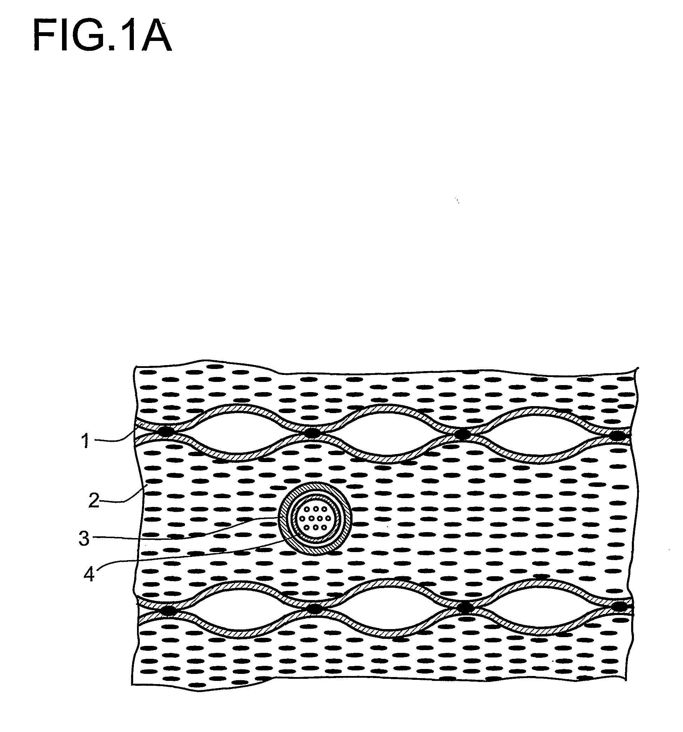

Figur 1 zeigt schematisch einen Ausschnitt aus einem Reaktor mit Thermoblechplatten 1 mit dazwischen angeordnetem Spalt 2, in den das Katalysatorfestbett eingebracht ist. Im Spalt 2 ist, in der dargestellten bevorzugten Ausführungsform, mittig eine Hülse 3 angeordnet, die ein Thermoelement 4 umhüllt, das beispielhaft 4 Messpunkte aufweist. Die Hülse 3 und das Thermoelement 4 ragen über einen Stutzen am Reaktormantel aus dem Reaktor heraus.

Die Querschnittsdarstellung in Figur 1A verdeutlicht die kreiszylindrische Geometrie der Hülse 3 mit darin angeordnetem Thermoelement 4.

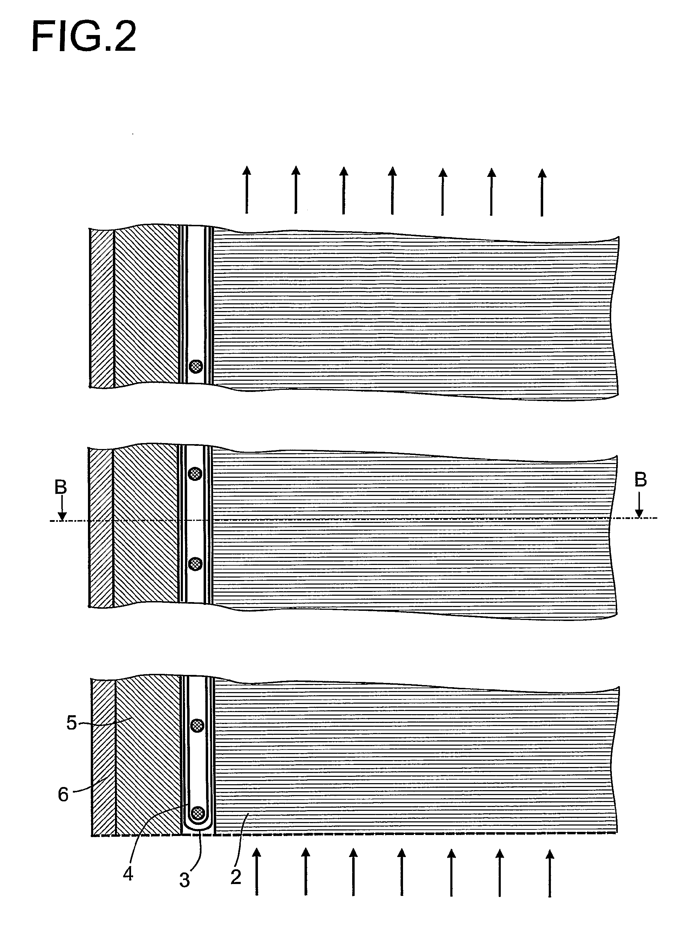

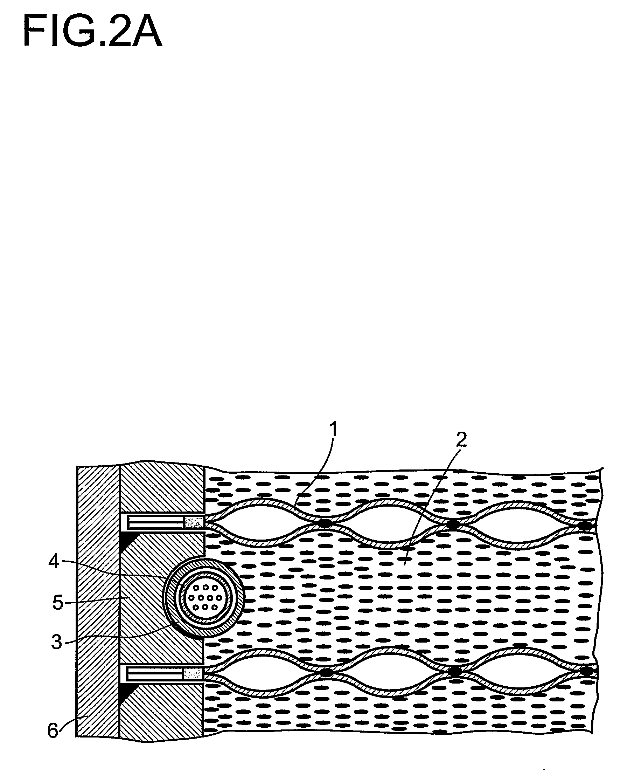

Die schematische Darstellung in Figur 2 zeigt einen Ausschnitt aus einem Reaktor in Längsrichtung, im Bereich eines Spaltes 2 zwischen zwei nicht dargestellten Thermoblechplatten. Im Spalt 2 ist, an der seitlichen Begrenzung 6 desselben, eine Hülse 3 mit Thermoelement 4 angeordnet. Zwischen Hülse 3 und seitlicher Begrenzung des Spaltes 2 ist eine Isolierkörper 5 vorgesehen.

Die Querschnittsdarstellung in Figur 2 verdeutlicht die Thermoblechplatten 1, einschließlich deren Befestigung an der seitlichen Begrenzung 6, sowie die kreiszylindrische Ausbildung der Hülse 3 mit Thermoelement 4 und formschlüssiger Ausbildung des Isolierkörpers 5.

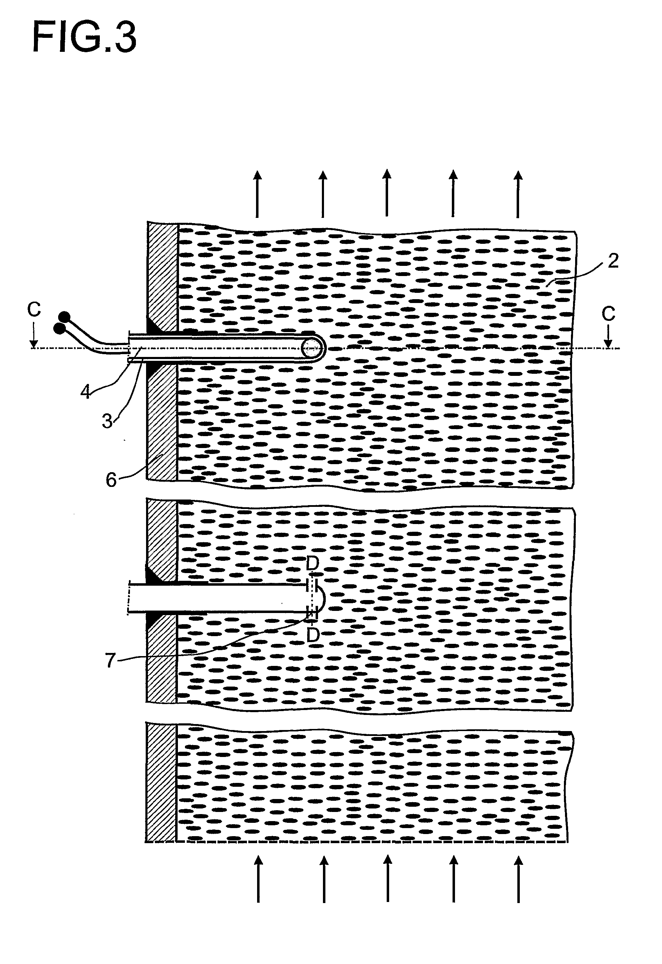

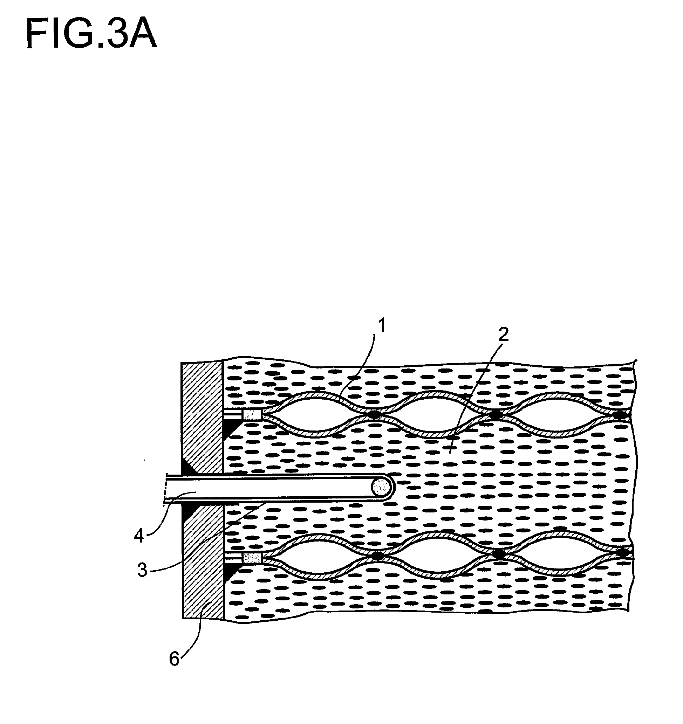

Figur 3 zeigt schematisch einen Ausschnitt aus einer weiteren Ausführungsform, mit horizontaler Anordnung einer Hülse 3 mit Thermoelement 4 in einem Spalt 2. Die Hülse weist in der Nähe ihres in den Spalt hineinragenden Endes Perforationen 7 auf, durch die Proben des Reaktionsgemisches abgezogen werden können.

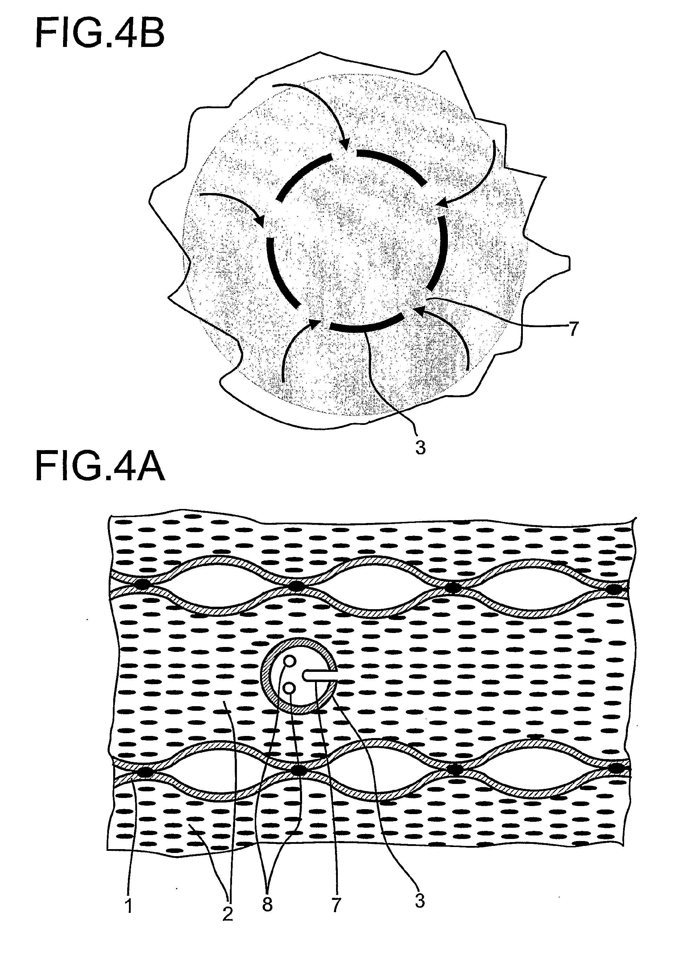

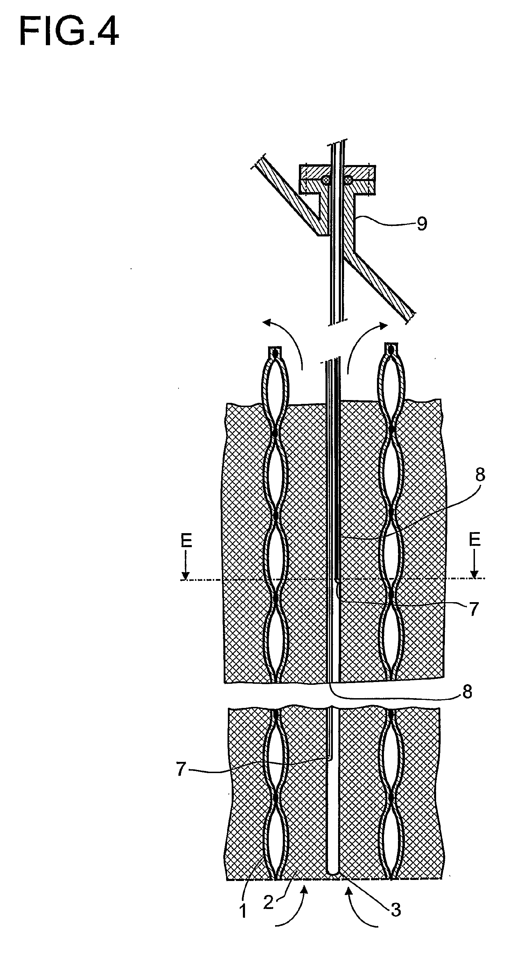

Die schematische Darstellung in Figur 4 zeigt einen Längsschnitt durch eine weitere Ausführungsform mit einer Hülse 3 mit Perforationen 7 in der Hülse 3 zur Aufnahme von Proben in die Probenahmeröhrchen 8. Die Hülse 3 mit Probenahmeröhrchen 8 ragen über den Stutzen 9 aus dem Reaktor hinaus.

Die Querschnittsdarstellung in Figur 4A verdeutlicht die Ausgestaltung der Hülse 3 im Querschnitt, mit Öffnung 7 und Probenahmeröhrchen 8.

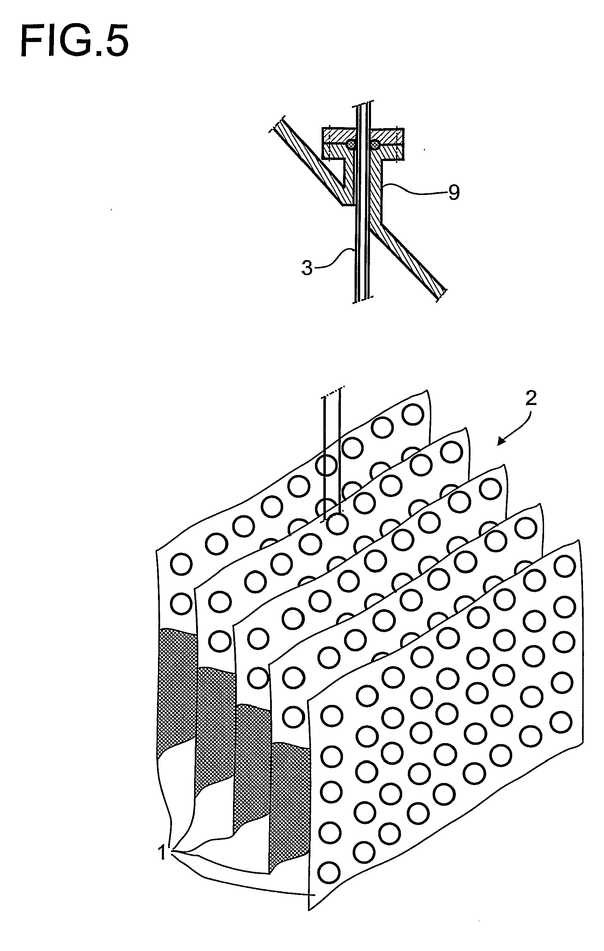

Figur 5 zeigt schematisch einen Ausschnitt aus einem Reaktor mit parallel angeordneten Thermoblechplatten 1, mit dazwischen liegenden Spalten 2. Beispielhaft ist eine Hülse 3 dargestellt, die in einen Spalt 2 zwischen zwei Thermoblechplatten 1, in Längsrichtung desselben, hineinragt, und die über einen Stutzen 9 am Reaktormantel außerhalb des Reaktors mündet.

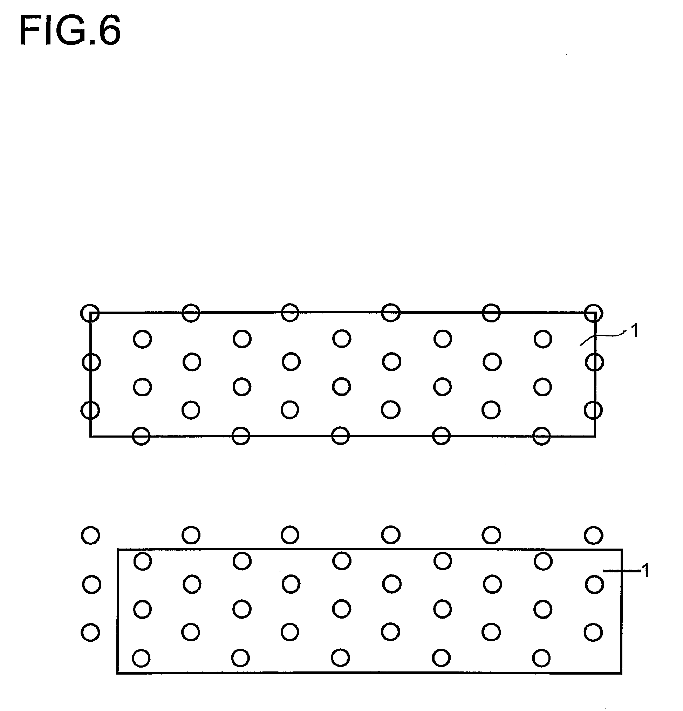

Figur 6 zeigt zwei bevorzugte Schweißpunktverteilungen auf der Oberfläche von Thermoblechplatten: dargestellt ist jeweils ein rechteckiger Oberflächenteilbereich einer Thermoblechplatte 1, entsprechend dem fünffachen Schweißpunktabstand auf der horizontalen Achse und dem fünffachen Reihenabstand auf der vertikalen Achse. Die obere Darstellung in Figur 6 zeigt eine bevorzugte Schweißpunktverteilung mit insgesamt 33 Schweißpunkten auf dem dargestellten Oberflächenteilbereich einer Thermoblechplatte 1 mit dem fünffachen Schweißpunktabstand und dem fünffachen Reihenabstand und die untere Darstellung eine weitere bevorzugte Anordnung mit 25 Schweißpunkten auf einem Oberflächenteilbereich gleicher Abmessung.

## Patentansprüche

1. Verfahren zur Herstellung von (Meth)acrolein und/oder (Meth)acrylsäure durch Partialoxidation von C₃- und/oder C₄-Vorläuferverbindungen in der Gasphase in Gegenwart eines heterogenen partikelförmigen Katalysators, in einem Reaktor mit zwei oder mehreren, vertikal, parallel zueinander unter Freilassung jeweils eines Spaltes (2) angeordneten Thermoblechplatten (1), wobei in den Spalten (2) der heterogene partikelförmige Katalysator eingebracht ist und das gasförmige Reaktionsgemisch durch die Spalte (2) geleitet wird, **dadurch gekennzeichnet, dass** man das Verfahren überwacht, steuert und/oder regelt, indem man als Überwachungs-, Steuerungs- und/oder Regelgröße einen oder mehrere Temperaturwerte wählt, die man in einem oder mehreren Spalten (2), an einer oder mehreren Messstellen, die über die Höhe jedes Spaltes (2) verteilt angeordnet sind, misst, unter Nutzung einer Vorrichtung mit einer Hülse (3), die im Spalt (2) angeordnet ist, die außerhalb des Reaktors mündet und die jeweils einen Temperaturmesseinsatz (4) umhüllt, der ein Multithermoelement ist.

2. Verfahren nach Anspruch 1, **dadurch gekennzeichnet, dass** man als weitere Überwachungs-, Steuerungs- und/oder Regelgröße die Zusammensetzung des gasförmigen Reaktionsgemisches in einem oder mehreren Spalten (2) wählt, die man an einer oder mehreren Messstellen, die über die Höhe jedes Spaltes (2) verteilt angeordnet sind, bestimmt.

3. Verfahren nach Anspruch 1, **dadurch gekennzeichnet, dass** die Hülse in Längsrichtung im Spalt (2) angeordnet ist.

4. Verfahren nach Anspruch 1 oder 3, **dadurch gekennzeichnet, dass** die Thermoblechplatten (1) in
- einem oder mehreren quaderförmigen Thermoblechplattenmodulen (10) angeordnet sind, die jeweils aus zwei oder mehreren rechteckigen, parallel zueinander unter Freilassung jeweils eines Spaltes (2) angeordneten Thermoblechplatten (1) gebildet sind, dass
- die Thermoblechplattenmodule (10) mit einer druckentlastenden, überwiegend zylinderförmigen Hülle (11, 12, 13), umfassend einen Zylindermantel (11) und denselben an beiden Enden abschließenden Hauben (12, 13) und deren Längsachse parallel zur Ebene der Thermoblechplatten (1) ausgerichtet ist, vollständig umgeben sind, dass
- ein oder mehrere Abdichtelemente (14, 15) dergestalt angeordnet sind, dass das gasförmige Reaktionsgemisch außer durch die von den Hauben (12, 13) begrenzten Reaktorinnenräume nur durch die Spalte (2) strömt und dass
- jedes Thermoblechplattenmodul (10) mit einem oder mehreren voneinander unabhängigen Temperaturmesseinsätzen (4), bevorzugt mit zwei oder drei, besonders bevorzugt mit drei Temperaturmesseinsätzen (4) ausgestattet ist.

5. Verfahren nach einem der Ansprüche 1, 3 oder 5, **dadurch gekennzeichnet, dass** die Hülse ein bevorzugt metallisches Rohr ist, insbesondere mit einem Außendurchmesser im Bereich von 4 bis 15 mm, bevorzugt im Bereich von 6 bis 10 mm, besonders bevorzugt im Bereich von 6 bis 8 mm und weiter bevorzugt mit einer Wandstärke von 0,8 bis 1,5 mm, bevorzugt von 1 mm.

6. Verfahren nach einem der Ansprüche 1 oder 3 bis 5, **dadurch gekennzeichnet, dass** die Hülse (3) eine Trennstelle innerhalb des Reaktorinnenraumes aufweist.

7. Verfahren nach einem der Ansprüche 1 oder 3 bis 6, **dadurch gekennzeichnet, dass** die Messstellen des Temperaturmesseinsatzes (4) in Reaktorbereichen mit zu erwartenden Temperaturextrema und/oder besonders großer Temperaturgradienten mit geringerem Abstand zueinander angeordnet sind und in den übrigen Reaktorbereichen mit größerem Abstand zueinander angeordnet sind.

8. Verfahren nach einem der Ansprüche 1 oder 3 bis 7, **dadurch gekennzeichnet, dass** der Temperaturmesseinsatz (4) 5 bis 60 Messstellen, bevorzugt 10 bis 50, besonders bevorzugt 15 bis 40 und weiter bevorzugt 20 bis 30 Messstellen aufweist.

9. Verfahren nach Anspruch 8, **dadurch gekennzeichnet, dass** der Temperaturmesseinsatz (4) 20 Messstellen und einen Außendurchmesser von etwa 3,8 mm aufweist und dass die Hülse (3) einen Außendurchmesser von 6 mm oder von 1/4 Zoll und einen Innendurchmesser von 4 mm oder von 5/32 Zoll aufweist.

10. Verfahren nach Anspruch 8, **dadurch gekennzeichnet, dass** der Temperaturmesseinsatz (4) 40 Messstellen und einen Außendurchmesser von etwa 2,5 mm aufweist und dass die Hülse (3) einen Außendurchmesser von 5 mm oder von 3/16 Zoll und einen Innendurchmesser von 3 mm oder von 1/8 Zoll aufweist.

11. Verfahren nach einem der Ansprüche 3 bis 10, **dadurch gekennzeichnet, dass** die Hülse (3) mittig in Längsrichtung im Spalt (2) angeordnet ist.

12. Verfahren nach einem der Ansprüche 3 bis 10, **dadurch gekennzeichnet, dass** die Hülse (3) an der seitlichen Begrenzung (6) des Spaltes (2) angeordnet ist.

13. Verfahren nach Anspruch 12, **dadurch gekennzeichnet, dass** zwischen der seitlichen Begrenzung (6) des Spaltes (2) und der Hülse (3) ein Isolierkörper vorgesehen ist, dass die Hülse (3) bevorzugt fest im Spalt (2) eingebaut ist und dass die Hülse (3) weiter bevorzugt einen Quadrat- oder Halbkreisquerschnitt aufweist.

14. Verfahren nach einem der Ansprüche 1 oder 4 bis 10, **dadurch gekennzeichnet, dass** die Hülse (3) horizontal im Spalt (2) angeordnet ist.

15. Verfahren nach Anspruch 2, **dadurch gekennzeichnet, dass** man für das Verfahren zusätzlich zu der in einem der Ansprüche 3 bis 16 definierten Vorrichtung jeweils eine Hülse (3) in einem oder mehreren Spalten (2) nutzt, die Perforationen (7) aufweist sowie mindestens ein Probenahmeröhrchen (8) zum Einführen in die Hülse (3), das in der Hülse (3) dergestalt angeordnet ist, dass das gasförmige Reaktionsgemisch über die Perforationen (7) in der Hülse (3) in das Probenahmeröhrchen (8) einströmt und aus dem Probenahmeröhrchen (8) außerhalb des Reaktors abgezogen und analysiert wird.

16. Verfahren nach Anspruch 15, **dadurch gekennzeichnet, dass** das Probenahmeröhrchen (8) mit der Hülse (3) fest verbunden ist, dergestalt, dass eine Öffnung des Probenahmeröhrchens (8) unmittelbar an einer Perforation (7) der Hülse (3) angeordnet ist.

17. Verfahren nach Anspruch 15, **dadurch gekennzeichnet, dass** das Probenahmeröhrchen (8) drehbar in der perforierten Hülse (3) angeordnet ist und zwei oder mehrere, über seine Mantelfläche versetzt angeordnete Öffnungen aufweist, dergestalt, dass das gasförmige Reaktionsgemisch stets nur über eine der Öffnungen in das Probenahmeröhrchen (8) einströmt.

18. Verfahren nach Anspruch 17, **dadurch gekennzeichnet, dass** die Öffnungen des Probenahmeröhrchens (8) als Schlitze in der Längsrichtung desselben ausgebildet sind.

19. Verfahren nach einem der Ansprüche 15 bis 18, **dadurch gekennzeichnet, dass** jedes Probenahmeröhrchen (8) zwei oder mehrere, bevorzugt 2 bis 4, voneinander getrennte Kammern aufweist, mit jeweils einer Öffnung, in die das gasförmige Reaktionsgemisch über die Perforationen (7) in der Hülse (3) einströmt und wobei das gasförmige Reaktionsgemisch aus jeder Kammer getrennt abgezogen und analysiert wird.

20. Verfahren nach Anspruch 19, **dadurch gekennzeichnet, dass** die Kammern nebeneinander oder konzentrisch zueinander angeordnet sind.

21. Verfahren nach Anspruch 18 oder 19, **dadurch gekennzeichnet, dass** das mehrere Kammern aufweisende Probenahmeröhrchen (8) um seine Längsachse drehbar ausgebildet ist.

22. Verfahren nach einem der Ansprüche 15 bis 21, **dadurch gekennzeichnet, dass** zwei oder mehrere Probenahmeröhrchen (8) vorgesehen sind, die jeweils fest mit der Hülse (3) verbunden sind, dergestalt, dass die Öffnung jedes Probenahmeröhrchens (8) unmittelbar an eine Perforation (7) der Hülse (3) angeordnet ist und wobei die einzelnen Probenahmeröhrchen (8) auf jeweils unterschiedlicher Höhe im Spalt (2) münden.

23. Verfahren nach Anspruch 15, **dadurch gekennzeichnet, dass** die Hülse (3) selbst als Probenahmeröhrchen (8) ausgebildet ist.

24. Verfahren zum Einbau einer Vorrichtung nach einem der Ansprüche 1 oder 3 bis 14 und/oder nach einem der Ansprüche 17 bis 25 in einen Reaktor, **dadurch gekennzeichnet, dass** der Einbau der Vorrichtung(en) von derselben Seite des Reaktors erfolgt, wie die Zuführung des gasförmigen Reaktionsgemisches.

25. Verfahren nach Anspruch 24, **dadurch gekennzeichnet, dass** der Einbau der Vorrichtung(en) sowie die Zuführung des gasförmigen Reaktionsgemisches jeweils von oben in den Reaktor erfolgen und dass die Hülse (3) lediglich im oberen Bereich des Spaltes (2) Perforationen (7) aufweist, insbesondere bis etwa zur Mitte des Spaltes (2).

26. Verfahren nach Anspruch 24, **dadurch gekennzeichnet, dass** der Einbau der Vorrichtung(en) sowie die Zuführung des gasförmigen Reaktionsgemisches in den Reaktor jeweils von unten erfolgen und dass bevorzugt durch die Thermoblechplatten (1) ein Wärmeträger geleitet wird, der unter Reaktionsbedingungen partiell oder vollständig verdampft.

## Claims

1. A process for preparing (meth)acrolein and/or (meth)acrylic acid by partially oxidizing C3 and/or C4 precursor compounds in the gas phase in the presence of a heterogeneous particulate catalyst, in a reactor having two or more thermoplates (1) arranged vertically and parallel to each other while in each case leaving a gap (2), the heterogeneous particulate catalyst being installed in the gaps (2) and the gaseous reaction mixture being passed through the gaps (2), which comprises monitoring, controlling and/or regulating the process by selecting as a monitoring, control and/or regulation parameter one or more temperatures which are measured in one or more gaps (2), at one or more measurement points which are distributed over the height of each gap (2), utilizing an apparatus having a sleeve (3) which is disposed in the gap (2) and opens outside the reactor and in each case encloses one temperature measurement insert (4) which is a multithermocouple.

2. The process according to claim 1, wherein the composition of the gaseous reaction mixture in one or more gaps (2) is selected as a further monitoring, control and/or regulation parameter and is determined at one or more measurement points which are distributed over the height of each gap (2).

3. The process according to claim 1, wherein the sleeve is disposed in the gap (2) in the longitudinal direction.

4. The process according to claim 1 or 3, wherein the thermoplates (1) are disposed in
- one or more cuboidal thermoplate modules (10) which are each formed from two or more rectangular thermoplates (1) arranged parallel to each other while in each case leaving a gap (2),
- the thermoplate modules (10) are completely surrounded by a pressure-releasing, predominantly cylindrical shell (11, 12, 13), comprising a cylinder jacket (11) and hoods (12, 13) which close it at both ends and whose longitudinal axis is aligned parallel to the plane of the thermoplates (1),
- one or more sealing elements (14, 15) are arranged in such a way that the gaseous reaction mixture, apart from flowing through the reactor interiors bounded by the hoods (12, 13), only flows through the gaps (2), and
- each thermoplate module (10) having one or more mutually independent temperature measurement inserts (4) is equipped preferably with two or three, more preferably with three, temperature measurement inserts (4).

5. The process according to any of claims 1, 3 and 5, wherein the sleeve is a preferably metallic tube, especially having an external diameter in the range from 4 to 15 mm, preferably in the range from 6 to 10 mm, more preferably in the range from 6 to 8 mm, and further preferably having a wall thickness of from 0.8 to 1.5 mm, preferably of 1 mm.

6. The process according to any of claims 1 and 3 to 5, wherein the sleeve (3) has a disconnection point within the reactor interior.

7. The process according to any of claims 1 and 3 to 6, wherein the measurement points of the temperature measurement insert (4) are arranged with a relatively small separation from each other in reactor regions having expected temperature extremes and/or particularly large temperature gradients, and with a relatively large separation from each other in the remaining reactor regions.

8. The process according to any of claims 1 and 3 to 7, wherein the temperature measurement insert (4) has from 5 to 60 measurement points, preferably from 10 to 50, more preferably from 15 to 40 and still more preferably from 20 to 30, measurement points.

9. The process according to claim 8, wherein the temperature measurement insert (4) has 20 measurement points and an external diameter of about 3.8 mm, and the sleeve (3) has an external diameter of 6 mm or of 1/4 inch and an internal diameter of 4 mm or of 5/32 inch.

10. The process according to claim 8, wherein the temperature measurement insert (4) has 40 measurement points and an external diameter of about 2.5 mm, and the sleeve (3) has an external diameter of 5 mm or of 3/16 inch and an internal diameter of 3 mm or of 1/8 inch.

11. The process according to any of claims 3 to 10, wherein the sleeve (3) is disposed in the gap (2) centrally in the longitudinal direction.

12. The process according to any of claims 3 to 10, wherein the sleeve (3) is disposed at the lateral boundary (6) of the gap (2).

13. The process according to claim 12, wherein an insulation element is provided between the lateral boundary (6) of the gap (2) and the sleeve (3), the sleeve (3) is preferably installed in the gap (2) in a fixed manner, and the sleeve (3) more preferably has a square or semicircular section.

14. The process according to any of claims 1 and 4 to 10, wherein the sleeve (3) is disposed horizontally in the gap (2).

15. The process according to claim 2, which, in addition to the apparatus defined in any of claims 3 to 16, utilizes in each case one sleeve (3) in one or more gaps (2) which has perforations (7) and also at least one sampling tube (8) for introduction into the sleeve (3), said sampling tube being disposed in the sleeve (3) in such a way that the gaseous reaction mixture flows through the perforations (7) in the sleeve (3) into the sampling tube (8) and is removed from the sampling tube (8) outside the reactor and analyzed.

16. The process according to claim 15, wherein the sampling tube (8) is connected to the sleeve (3) in a fixed manner, in such a way that an orifice of the sampling tube (8) is disposed directly on a perforation (7) of the sleeve (3).

17. The process according to claim 15, wherein the sampling tube (8) is disposed in the perforated sleeve (3) in a rotatable manner and has two or more orifices disposed over its jacket surface offset in such a way that the gaseous reaction mixture always flows into the sampling tube (8) only through one of the orifices.

18. The process according to claim 17, wherein the orifices of the sampling tube (8) are designed as slots in the longitudinal direction thereof.

19. The process according to any of claims 15 to 18, wherein each sampling tube (8) has two or more, preferably from 2 to 4, mutually separate chambers, each having an orifice into which the gaseous reaction mixture flows through the perforations (7) in the sleeve (3), and the gaseous reaction mixture is removed separately from each chamber and analyzed.

20. The process according to claim 19, wherein the chambers are arranged mutually adjacently or concentrically.

21. The process according to claim 18 or 19, wherein the sampling tube (8) having a plurality of chambers is designed in a rotatable manner about its longitudinal axis.

22. The process according to any of claims 15 to 21, wherein two or more sampling tubes (8) are provided and are each connected in a fixed manner to the sleeve (3), in such a way that the orifice of each sampling tube (8) is disposed directly on a perforation (7) of the sleeve (3), and the individual sampling tubes (8) open in the gap (2) each at a different height.

23. The process according to claim 15, wherein the sleeve (3) is itself designed as a sampling tube (8).

24. A process for incorporating an apparatus according to any of claims 1 and 3 to 14 and/or according to any of claims 17 to 25 into a reactor, wherein the apparatus(es) is/are installed from the same side of the reactor as the feed of the gaseous reaction mixture.

25. The process according to claim 24, wherein the apparatus(es) is/are installed and the gaseous reaction mixture is fed into the reactor in each case from above, and the sleeve (3) has perforations (7) only in the upper region of the gap (2), especially up to about the middle of the gap (2).

26. The process according to claim 24, wherein the apparatus(es) is/are installed and the gaseous reaction mixture is fed into the reactor in each case from below, and a heat carrier is preferably passed through the thermoplates (1) and partially or fully evaporates under reaction conditions.

## Revendications

1. Procédé de fabrication de (méth)acroléine et/ou d'acide (méth)acrylique par oxydation partielle en phase gazeuse de composés précurseurs en C₃ et/ou en C₄ et en présence d'un catalyseur hétérogène en forme de particules dans un réacteur qui présente deux ou plusieurs plaques thermiques (1) disposées à la verticale et parallèlement les unes aux autres en laissant chaque fois entre elles un interstice (2), le catalyseur hétérogène en particules étant placé dans les interstices (2) et le mélange gazeux de réaction étant amené dans l'interstice (2),
**caractérisé en ce que**
on surveille, commande et/ou régule le procédé en sélectionnant comme grandeur de surveillance, de commande et/ou de régulation une ou plusieurs valeurs de température que l'on mesure dans un ou plusieurs interstices (2) en un ou plusieurs emplacements de mesure répartis sur la hauteur de chaque interstice (2) en recourant à un dispositif doté d'une douille (3) disposée dans l'interstice (2), qui débouche à l'extérieur du réacteur et qui entoure chaque fois une garniture (4) de mesure de température qui est un multithermoélément.

2. Procédé selon la revendication 1, **caractérisé en ce que** comme autre grandeur de surveillance, de commande et/ou de régulation, on sélectionne la composition du mélange gazeux de réaction dans un ou plusieurs interstices (2), composition que l'on détermine en un ou plusieurs emplacements de mesure répartis sur la hauteur de chaque interstice (2).

3. Procédé selon la revendication 1, **caractérisé en ce que** la douille est disposée dans le sens de la longueur de l'interstice (2).

4. Procédé selon les revendications 1 ou 3, **caractérisé en ce que** les plaques thermiques (1) sont disposées
en un ou plusieurs modules (10) en forme de parallélépipède de plaques thermiques qui sont formés chacun de deux ou plusieurs plaques thermiques (1) rectangulaires, parallèles les unes aux autres et disposées de manière à laisser entre elles un interstice (2),
**en ce que** les modules (10) de plaques thermiques sont complètement entourés par une enveloppe (11, 12, 13) de délestage de pression, de forme essentiellement cylindrique, comprenant une enveloppe cylindrique (11) fermée par des capots (12, 13) à ses deux extrémités et dont l'axe longitudinal est orienté parallèlement au plan des plaques thermiques (1),
**en ce qu'**un ou plusieurs éléments d'étanchéité (14, 15) sont disposés de telle sorte que le mélange gazeux de réaction ne s'écoule que dans les interstices (2) en plus des espaces intérieurs du réacteur délimités par les capots (12, 13) et
**en ce que** chaque module (10) de plaques thermiques est équipé d'une ou plusieurs garnitures (4) de mesure de température indépendantes les unes des autres, de préférence de deux ou trois et de façon particulièrement préférable de trois garnitures (4) de mesure de température.

5. Procédé selon l'une des revendications 1, 3 ou 5, **caractérisé en ce que** la douille est de préférence un tube métallique, en particulier d'un diamètre extérieur de l'ordre de 4 à 15 mm, de préférence de l'ordre de 6 à 10 mm, de façon particulièrement préférable de l'ordre de 6 à 8 mm et de façon encore plus préférable dont la paroi a une épaisseur de 0,8 à 1,5 mm et de préférence de 1 mm.

6. Procédé selon l'une des revendications 1 ou 3 à 5, **caractérisé en ce que** la douille (3) présente un emplacement de séparation à l'intérieur de l'espace intérieur du réacteur.

7. Procédé selon l'une des revendications 1 ou 3 à 6, **caractérisé en ce que** les emplacements de mesure de la garniture (4) de mesure de température dans les parties du réacteur dans lesquels on s'attend à des extrémums de températures et/ou à des gradients de températures particulièrement élevés sont disposés à plus petite distance les uns des autres et dans les autres parties du réacteur, sont disposés à plus grande distance les uns des autres.

8. Procédé selon l'une des revendications 1 ou 3 à 7, **caractérisé en ce que** la garniture (4) de mesure de température présente de 5 à 60 emplacements de mesure, de préférence de 10 à 50, de façon particulièrement préférable de 15 à 40 et de façon encore plus préférable de 20 à 30 emplacements de mesure.

9. Procédé selon la revendication 8, **caractérisé en ce que** la garniture (4) de mesure de température présente 20 emplacements de mesure et un diamètre extérieur de 3,8 mm et **en ce que** la douille (3) présente un diamètre extérieur de 6 mm ou de 1/4 pouce et un diamètre intérieur de 4 mm ou de 5/32 pouce.

10. Procédé selon la revendication 8, **caractérisé en ce que** la garniture (4) de mesure de température présente 40 emplacements de mesure et un diamètre extérieur d'environ 2,5 mm et **en ce que** la douille (3) présente un diamètre extérieur de 5 mm ou de 3/16 pouce et un diamètre intérieur de 3 mm ou de 1/8 pouce.

11. Procédé selon l'une des revendications 3 à 10, **caractérisé en ce que** la douille (3) est disposée au milieu du sens de la longueur de l'interstice (2).

12. Procédé selon l'une des revendications 3 à 10, **caractérisé en ce que** la douille (3) est disposée sur la frontière latérale (6) de l'interstice (2).

13. Procédé selon la revendication 12, **caractérisé en ce qu'**entre la frontière latérale (6) de l'interstice (2) et la douille (3) est prévu un corps isolant et **en ce que** la douille (3) est montée de préférence de manière fixe dans l'interstice (2) et **en ce que** la douille (3) présente de façon encore plus préférable une section transversale en carré ou en demi-cercle.

14. Procédé selon l'une des revendications 1 ou 4 à 10, **caractérisé en ce que** la douille (3) est disposée horizontalement dans l'interstice (2).

15. Procédé selon la revendication 2, **caractérisé en ce que** pour le procédé, en plus de l'ensemble défini dans l'une des revendications 3 à 16, on utilise dans un ou plusieurs interstices (2) une douille (3) qui présente des perforations (7) ainsi qu'au moins un tube (8) de prélèvement d'échantillon destiné à être inséré dans la douille (3) et disposé dans le douille (3) de telle sorte que le mélange gazeux de réaction pénètre par les perforations (7) ménagées dans la douille (3) dans le tube (8) de prélèvement d'échantillon et soit extrait hors du réacteur par le tube (8) de prélèvement d'échantillon et analysé.

16. Procédé selon la revendication 15, **caractérisé en ce que** le tube (8) de prélèvement d'échantillon est raccordé solidairement à la douille (3) de telle sorte qu'une ouverture du tube (8) de prélèvement d'échantillon soit disposée directement sur une perforation (7) de la douille (3).

17. Procédé selon la revendication 15, **caractérisé en ce que** le tube (8) de prélèvement d'échantillon est disposé à rotation dans la douille perforée (3) et présente deux ou plusieurs ouvertures disposées sur sa surface d'enveloppe, de telle sorte que le mélange gazeux de réaction pénètre toujours dans le tube (8) de prélèvement d'échantillon uniquement par une des ouvertures.

18. Procédé selon la revendication 17, **caractérisé en ce que** les ouvertures du tube (8) de prélèvement d'échantillon sont configurées comme fentes orientées dans le sens de sa longueur.

19. Procédé selon l'une des revendications 15 à 18, **caractérisé en ce que** chaque tube (8) de prélèvement d'échantillon présente deux ou plusieurs chambres, de préférence de 2 à 4 chambres, séparées les unes des autres et dotées chacune d'une ouverture dans laquelle le mélange gazeux de réaction pénètre dans la douille (3) par les perforations (7), le mélange gazeux de réaction étant extrait séparément de chaque chambre avant d'être analysé.

20. Procédé selon la revendication 19, **caractérisé en ce que** les chambres sont disposées les unes à côté des autres ou concentriquement les unes par rapport aux autres.

21. Procédé selon les revendications 18 ou 19, **caractérisé en ce qu'**un tube (8) de prélèvement d'échantillon présentant plusieurs chambres est configuré de manière à pouvoir tourner autour de son axe longitudinal.

22. Procédé selon l'une des revendications 15 à 21, **caractérisé en ce que** deux ou plusieurs tubes (8) de prélèvement d'échantillon sont prévus, sont chacun raccordé solidairement à la douille (3) de telle sorte que l'ouverture de chaque tube (8) de prélèvement d'échantillon soit disposée directement sur une perforation (7) de la douille (3), les différents tubes (8) de prélèvement d'échantillon débouchant à des hauteurs différentes dans l'interstice (2).

23. Procédé selon la revendication 15, **caractérisé en ce que** la douille (3) est elle-même configurée comme tube (8) de prélèvement d'échantillon.

24. Procédé de montage d'un ensemble selon l'une des revendications 1 ou 3 à 14 et/ou selon l'une des revendications 17 à 25 dans un réacteur, **caractérisé en ce que** le montage du ou des ensembles s'effectue du côté du réacteur par lequel le mélange gazeux de réaction est apporté.

25. Procédé selon la revendication 24, **caractérisé en ce que** le montage du ou des ensembles ainsi que l'amenée du mélange gazeux de réaction dans le réacteur s'effectuent par le haut et **en ce que** la douille (3) ne présente de perforation (7) que dans la partie supérieure de l'interstice (2), en particulier sensiblement jusqu'au milieu de l'interstice (2).

26. Procédé selon la revendication 24, **caractérisé en ce que** le montage du ou des ensembles ainsi que l'amenée du mélange gazeux de réaction dans le réacteur s'effectuent par le bas et **en ce qu'**un caloporteur qui se vaporise partiellement ou complètement dans les conditions de réaction est apporté de préférence par les plaques thermiques (1).
